# DEMANDE DE BREVET EUROPEEN

(11) **EP 0 998 909 A1**
(43) Date de publication de la demande: **10.05.2000**
(21) Numéro de dépôt: 99401644.2
(22) Date de dépôt: 01.07.1999
(51) Int. Cl.: A61K 7/38

(54) **Composition déodorante**

(30) Priorité: 13.10.1998 FR 9812802
(71) Demandeur: L'OREAL, 75008 Paris (FR)
(72) Inventeur: Ribery, Delphine, 92300 Levallois-Perret (FR); Aubert, Lionnel, 95330 Domont (FR)
(74) Mandataire: Miszputen, Laurent

(57) **Abrégé**

La présente invention concerne une composition déodorante sous forme d'émulsion eau-dans-huile comprenant une quantité efficace d'au moins un sel d'alun à l'état dissous dans la phase aqueuse de ladite composition.

La présente invention concerne également un procédé pour traiter les odeurs axillaires humaines à l'aide de la présente composition déodorante.

## Description

La présente invention concerne une composition déodorante sous forme d'émulsion eau-dans-huile comprenant une quantité efficace d'au moins d'un sel d'alun à l'état dissous dans la phase aqueuse de ladite composition.

La présente invention concerne également un procédé de désodorisation mettant en oeuvre ladite composition et plus spécialement un procédé pour traiter les odeurs axillaires humaines consistant à appliquer sur la surface axillaire une quantité efficace de ladite composition.

Dans le domaine cosmétique, il est bien connu d'utiliser en application topique, des produits déodorants contenant des substances actives de type antitranspirant ou de type bactéricide pour diminuer voire supprimer les odeurs axillaires généralement désagréables.

Les substances antitranspirantes ont pour effet de limiter le flux sudoral. Elles sont généralement constituées de sels d'aluminium qui, d'une part, sont irritants pour la peau et qui, d'autre part, diminuent le flux sudoral en modifiant la physiologie cutanée, ce qui n'est pas satisfaisant.

Les substances bactéricides inhibent le développement de la flore cutanée responsable des odeurs axillaires. Parmi les produits bactéricides, le plus couramment employé est le Triclosan (5-chloro-2-(2,4-dichlorophénoxy) phénol), qui présente l'inconvénient de modifier de façon importante l'écologie de la flore cutanée, d'être inhibé par certains composés, comme par exemple les tensioactifs non-ioniques, couramment utilisés dans la formulation de compositions cosmétiques. Le caractère insoluble du Triclosan dans l'eau ne permet pas son incorporation dans des formules essentiellement aqueuses.

Dans le but d'obtenir une efficacité à long terme, on recherche des produits exerçant une action d'actif déodorant, c'est-à-dire des produits qui soient capables de modifier, de réduire et/ou d'ôter ou de prévenir le développement des odeurs corporelles (cette définition est donnée dans l'ouvrage « Cosmetic Science and Technology Séries » - 1988 / Volume 7 chap.10 - Illc). En outre, on cherche des produits qui ne présentent pas les inconvénients des substances actives utilisées dans l'art antérieur.

On sait que certains sels d'aluminium ont des bonnes propriétés déodorantes et sont mieux tolérés que les actifs cités précédemment. C'est le cas par exemple des aluns comme l'alun de potassium ou l'alun d'ammonium qui sont connus depuis longtemps comme actifs bactéricides et astringents.

Différents types de formulations ont été mises en oeuvre à base de ces actifs depuis de nombreuses années.

On connaît dans les brevets EP-B-157736 et US 5,399,364 des compositions déodorantes moulées à partir de pierres d'alun à l'état brut. Ces formulations sont peu confortables à l'application, sont difficiles à étaler sur la peau et nécessitent avant application des mouillages fréquents à l'eau pour obtenir une faible efficacité. D'autre part, ces produits ne permettent pas d'introduire d'autres additifs cosmétiques.

On connaît également dans l'état de la technique des compositions déodorantes anhydres se présentant sous forme de poudres, de dispersions anhydres contenant un sel d'alun sous forme sèche ou non-solubilisée telles que celles décrites dans les demandes de brevet JP04103519, JP60239411, EP-A-0593657, JP58057313 et EP-A-0091368. On connaît aussi dans la demande de brevet W096/00566 des sticks moulés à partir d'alun pur broyé et d'un plastifiant tel que le glycérol ou le sorbitol. Ce type de composition présente l'inconvénient d'être peu confortable à l'utilisation par manque de sensation de fraîcheur. D'autre part, ces formulations à base d'alun sous forme de poudre sont difficiles à réaliser en raison d'une homogénéité difficile à maîtriser.

Pour pallier à ces inconvénients, on a réalisé des formulations aqueuses déodorantes dans lesquelles l'alun est dissous dans la phase aqueuse. On connaît notamment des solutions aqueuses ou hydroalcooliques à base d'alun de potassium dans les demandes de brevet FR-A-2551658, CN1087261, FR-A-2575922, FR-A-2201880. On connaît dans les demandes de brevet japonais Hei 2-111714, Sho 59-36607, Sho-58-83612, Hei 3-110013 également des émulsions huile-dans-eau contenant de l'alun de potassium ou d'ammonium à l'état dissous dans la phase aqueuse. On connaît également dans les demandes de brevet japonais Hei 2-31688 et Sho 61-1002 des formulations à trois phases non-miscibles pouvant contenir de l'alun dissous dans une phase aqueuse.

Toutes ces formulations déodorantes aqueuses dans lesquelles l'alun est dissous présentent l'inconvénient de ne pas pouvoir utiliser ce type d'actif à des concentrations supérieures à sa solubilité dans l'eau pour obtenir une efficacité déodorante optimale. Or les aluns les plus couramment utilisés en cosmétique comme l'alun de potassium ou l'alun d'ammonium possèdent une faible solubilité dans l'eau (ie : respectivement 11,4% en poids et 12% en poids à 20°C selon l'ouvrage « Encyclopedia of Chemical Technology ; 4ème édition volume 2 ; pages 330-337 ») et ne peuvent être utilisés dans ces compositions déodorantes aqueuses au-delà de cette quantité à partir de laquelle on observe durant le stockage selon les tests classiques de conservation des produits cosmétiques (ie : après 2 mois de conservation à 4°C, à température ambiante et à 45°C) un phénomène de recristallisation indésirable. Cette recristallisation a pour conséquence de produire des produits cosmétiques d'aspect hétérogène.

La demanderesse a découvert de manière surprenante que les supports du type émulsion eau-dans-huile pouvaient contenir tous les aluns et plus particulièrement ceux de faible solubilité dans l'eau à l'état dissous dans la phase aqueuse à des concentrations supérieures à leur solubilité sans observer de phénomène de recristallisation de l'alun durant leur stockage selon les tests classiques de conservation des produits cosmétiques.

Cette découverte est à la base de l'invention.

Ainsi, conformément à l'un des objets de la présente invention, il est maintenant proposé de nouvelles compositions déodorantes, qui sont essentiellement caractérisées par le fait qu'elles se présentent sous la forme d'une émulsion eau-dans-huile contenant une quantité efficace d'au moins un alun à l'état dissous dans la phase aqueuse de ladite émulsion.

On entend par « quantité efficace en alun » une quantité suffisante d'alun, dans la forme galénique utilisée, pour produire après application un effet déodorant satisfaisant.

On entend par effet déodorant , une activité permettant de réduire sensiblement voire supprimer les mauvaises odeurs et plus particulièrement les odeurs corporelles.

Un autre objet de l'invention concerne également l'utilisation desdites compositions pour la fabrication de produits cosmétiques déodorants destinés à traiter les odeurs axillaires humaines.

Un autre objet de l'invention consiste en de nouvelles formulations cosmétiques ou dermatologiques déodorantes, qui sont essentiellement caractérisées par le fait qu'elles sont constituées par une composition telle que définie ci-dessus.

Un autre objet de l'invention consiste en un procédé de désodorisation mettant en oeuvre ladite composition, et plus particulièrement un procédé pour traiter les odeurs axillaires humaines, consistant à appliquer sur la surface axillaire une quantité efficace de ladite composition.

Un autre objet de l'invention est l'utilisation d'un support du type émulsion eau-dans-huile pour la fabrication de produits cosmétiques ou dermatologiques déodorants aqueux contenant au moins un sel d'alun à l'état dissous dans une quantité par rapport à l'eau située au-dessus de la solubilité dans l'eau dudit alun.

D'autres objets apparaîtront à la lumière de la description et des exemples qui suivent.

Conformément à la présente invention, les aluns utilisables sont choisis parmi notamment :
- l'alun de potassium de structure : KAl(SO₄)₂, 12H₂O
- l'alun d'ammonium de structure : NH₄Al(SO₄)₂, 12H₂O
- l'alun de sodium de structure : NaAl(SO₄)₂, 12H₂O
- les sels de sulfate d'aluminium de structure : Al₂(SO₄)₃, n H₂O où n vaut 0 à 27

Les compositions selon l'invention conviennent plus particulièrement pour les aluns de solubilité faible dans l'eau et notamment ayant une solubilité inférieure à 30 g/100 ml d'eau à 20°C et plus particulièrement une solubilité dans l'eau inférieure à 13 g/100 ml d'eau à 20°C tels que :
- l'alun de potassium (11,4 g/100 ml d'eau à 20°C),
- l'alun d'ammonium (12 g/100 ml d'eau à 20°C).

On utilisera plus particulièrement l'alun de potassium et notamment le produit commercial vendu par la Société GIULINI CHEMIE ( WARWICK CHIMILUX SA).

Les aluns sont présents à l'état dissous dans la phase aqueuse des compositions conformes à l'invention dans des concentrations allant de préférence entre 0,5 à 30% en poids par rapport au poids total de la composition. Bien entendu, la concentration de l'actif alun varie en fonction du choix de la forme galénique choisie. Elle peut varier de 1 à 20% en poids pour les formulations sous forme de sticks, de crèmes, de gels, de roll-on. Elle peut varier de 1 à 15% en poids pour les formulations conditionnées dans des dispositifs aérosol.

Selon une forme particulièrement préférée de l'invention, le sel d'alun sera présent dans la phase aqueuse dans une concentration supérieure à 10% en poids et plus préférentiellement supérieure ou égale 15% en poids.

Les compositions selon l'invention comprennent une phase grasse qui peut comporter un ou plusieurs corps gras constitués par une ou plusieurs huiles et/ou une ou plusieurs cires. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline), les huiles végétales (huile d'amande douce, de macadamia, de pépin de cassis, de jojoba), les huiles synthétiques comme le perhydrosqualène, les alcools, les acides ou les esters gras (comme le benzoate d'alcools en C₁₂-C₁₅ vendu sous la dénomination commerciale « Finsolv TN » par la société Finetex), le palmitate d'octyle, le lanolate d'isopropyle, les triglycérides dont ceux des acides caprique/caprylique, les esters et éthers gras oxyéthylénés ou oxypropylénés, les huiles fluorées ou perfluorées ou encore les polyalkylènes comme les polydécènes.

Comme composés cireux, on peut citer les cires animales, fossiles, végétales, minérales ou de synthèse, l'huile de ricin hydrogénée. On peut citer notamment les cires d'abeille, les cires de Carnauba, de Candelila, de canne à sucre, du Japon, les ozokérites, la cire de Montan, les cires microcristallines, les paraffines, les cires de polyéthylène, les cires et résines de silicone.

La phase grasse peut également comprendre une huile de silicone, volatile ou non telle que les cyclométhicones ou les diméthicones. On peut par exemple mettre en oeuvre dans les compositions de la présente invention une huile de silicone volatile comme par exemple les cyclométhicones vendues sous les dénominations commerciales «DC245 Fluid» ou « DC 246 Fluid » par Dow Corning.

La phase grasse est de préférence présente dans les compositions dans des concentrations pondérales allant de 5% à 80% par rapport au poids total de la composition, et de préférence dans des concentrations pondérales allant de 10% à 50% par rapport au poids total de la composition,

La phase aqueuse de ces émulsions peut renfermer des ingrédients autres que l'eau, tels que des humectants comme les polyols, des stabilisants comme l'acide citrique ou l'acide lactique, des séquestrants (acide éthylene diamine tétracétique).

De façon connue, l'émulsion selon l'invention peut contenir un ou plusieurs tensioactifs émulsionnants d'émulsion eau-dans-huile (que l'on nommera ci-après émulsionnants E/H). Comme émulsionnants E/H utilisables dans l'invention, on peut utiliser tout émulsionnant cosmétique ayant en général un HLB (balance hydrophile-lipophile) inférieur ou égal à 6. On peut citer par exemple les esters d'acide gras et de glucose tels que le dioléate de méthylglucose, les esters d'acide gras et de glycérine tels que l'isostéarate de glycéryle, l'oléate de glycéryle et le ricinoléate de glycéryle, les esters d'acide gras et de sorbitol tels que le tristéarate de sorbitan et le di- ou tri-oléate de sorbitan ; les polyalkyl polyéther siloxanes portant des groupes polyoxyalkylénés greffés sur la chaîne siliconée principale. On utilisera de façon préférentielle les polyalkylpolyéthersiloxanes portant des groupes polyoxyalkylénés greffés sur la chaîne siliconée principale.

Parmi les émulsionnants E/H siliconés, on utilisera plus particulièrement ceux choisis dans le groupe formé par les polydiorganosiloxanes de formules (I) et (II) suivantes, ces composés pouvant eux-mêmes être dispersés dans une diméthicone volatile, 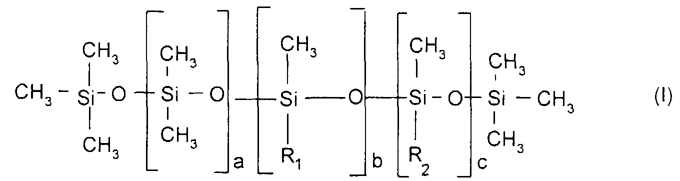 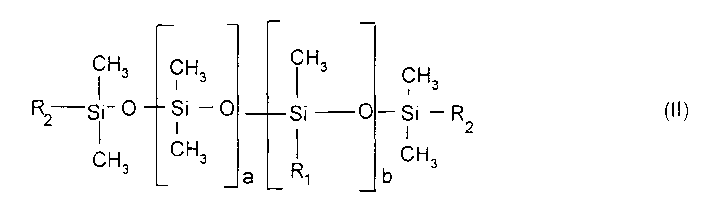 dans lesquelles :
R₁ désigne un groupement alkyle linéaire ou ramifié en C₁₂-C₂₀ et de préférence en C₁₂-C₁₈ ;
R₂ désigne le groupement :--CₙH₂ₙ--(-OC₂H₄-)ₓ--(-OC₃H₆-)_{y}--O―R₃,
R₃ désigne un atome d'hydrogène ou un radical alkyle linéaire ou ramifié comportant de 1 à environ 12 atomes de carbone,
a désigne un nombre entier allant de 0 à environ 500,
b est un nombre entier allant de 0 à environ 500
c désigne un nombre entier allant de 1 à environ 500,
n est un nombre entier allant de 2 à 12,
x et y désignent respectivement un nombre entier allant de 0 à environ 50, la somme x + y étant supérieure ou égale à la valeur 1.

Parmi les polyorganosiloxanes émulsionnants préférentiels, on peut citer ceux de formule (I) dans laquelle, a est un nombre entier allant de 2 à 450, b est égal à 0, c est un nombre entier allant de 2 à 40, n est un nombre entier allant de 2 à 5, x est un nombre entier allant de 1 à 30, y est un nombre entier allant de 0 à 30, avec x ≥ y. Parmi ces émulsionnants siliconés, on peut utiliser plus particulièrement le mélange de cyclométhicone et de diméthicone copolyol (nom CTFA.) tel que le produit vendu par la société Dow Corning sous la dénomination commerciale Silicone DC 3225 C ou le produit vendu par la société GOLDSCHMIDT sous le nom d'ABIL EM 97.

Parmi les polyorganosiloxanes émulsionnants préférentiels, on peut citer ceux de formule (I) dans laquelle a est égal à 0 et b est différent de 0 tels que plus particulièrement le laurylméthicone copolyol (nomenclature C.T.F.A. - 7ème édition - 1997) vendu par la société Dow Corning sous la dénomination commerciale Q2-5200 Formulation Aid ou bien le cétyl diméthicone copolyol qui est par exemple vendu sous les dénominations « ABIL WE09® » et « ABIL EM90® » par la société Goldschmidt.

Dans les compositions selon l'invention, le tensioactif émulsionnant E/H est présent de préférence dans des proportions pondérales allant d'environ 0,1% à environ 10%, et de préférence allant d'environ 1% à environ 8% par rapport au poids total de la composition.

Les compositions déodorantes de la présente invention peuvent comprendre en outre d'autres actifs déodorants classiques en plus de l'alun.

Ces actifs déodorants peuvent être choisis par exemple parmi : des sels hydrosolubles de zinc, comme par exemple le pyrrolidone carboxylate de zinc (plus communément appelé pidolate de zinc), le sulfate de zinc, le chlorure de zinc, le lactate de zinc, le gluconate de zinc et le phénolsulfonate de zinc ; des sels d'aluminium, comme par exemple le chlorure d'aluminium et les hydroxyhalogénures d'aluminium ; des sels de zirconium, comme par exemple des sels d'oxyde de zirconium, des sels d'hydroxyzirconyle ; des complexes de métaux comme l'aluminium ou le zirconium avec un acide aminé comme par exemple la glycine comme décrit dans US-3,792,068 ; des bactéricides.

Les compositions de l'invention peuvent comprendre en outre des adjuvants cosmétiques choisis parmi les corps gras, les solvants organiques, les gélifiants, les émollients, les adoucissants, les antioxydants, les opacifiants, les stabilisants, les silicones, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, les colorants, les pigments, les agents épaississants, ou tout autre ingrédient habituellement utilisé en cosmétique.

Les tensioactifs sont choisis de préférence parmi les tensioactifs non-ioniques, comme par exemple les produits de condensation d'un alcool gras ou d'un acide gras avec une chaine polyalkylèneglycol.

Les épaississants, de préférence non ioniques, peuvent être choisis parmi les gommes de guar et celluloses modifiées ou non telles que la gomme de guar hydroxypropylée, la cétylhydroxyéthylcellulose, les hectorites comme par exemple la Bentone Gel MiO vendue par la société NL INDUSTRIES, la Silice AEROSIL R 972 vendue par la société DEGUSSA.

Elles peuvent comprendre des émollients, qui contribuent à une sensation douce, sèche, non-collante à l'application de la composition sur la peau. Ces émollients peuvent être choisis parmi des produits du type silicone volatile, des silicones non-volatiles et d'autres émollients non-volatils.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones D4 ou D5.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 200 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les émollients non-volatils utilisables dans la présente invention, on peut citer par exemple : les dérivés hydrocarbonés, les huiles minérales, les alcools gras, les esters d'alcools en C₃-C₁₈ avec des acides en C₃-C₁₈, les esters de l'acide benzoïque avec des alcools en C₁₂-C₁₈ et leurs mélanges, des polyols en C₂-C₆ choisis de préférence parmi le glycérol, le propylèneglycol ou le sorbitol, les polymères de polalkylène glycol.

Lorsque les compositions déodorantes selon l'invention sont destinées à l'usage cosmétique, elles peuvent se présenter sous forme de lotions, de crèmes ou de gels fluides distribués en spray aérosol, en flacon pompe ou en roll-on, sous forme de crèmes épaisses distribuées en tubes et sous forme de sticks, et contenir à cet égard les ingrédients et propulseurs généralement utilisés dans ce type de produits et bien connus de l'homme de l'art, sous réserve qu'ils n'interfèrent pas avec les aluns décrits dans la présente invention.

L'invention peut aussi trouver des applications intéressantes dans le domaine des produits d'entretien et désodorisants divers (air ambiant, textiles, réfrigérateurs, vide-ordures, poubelles, litières et cages d'animaux domestiques ou gaines de ventilation des immeubles).

Des exemples concrets, mais nullement limitatifs, illustrant l'invention vont maintenant être donnés.

### Exemple 1 : stick déodorant selon l'invention

| **Ingrédients** | **% en poids** |
|---|---|
| Laurylméthicone copolyol vendu par la société Dow Corning | |
| Q2- 5200 Formulation Aid | 5,00 |
| Antioxydant | 0,05 |
| Cyclohexadiméthylsiloxane vendue par la société Dow Corning sous le nom de Dow Corning 246 fluid | 22,50 |
| Cire de polyéthylène vendue par la société Pétrolite sous le nom de POLYWAX 500 POLYETHYLENE | 12,5 |
| Alun de potassium vendu par la Société GIULINI CHEMIE (Warwick Chimilux SA) | 15,00 (soit 33,3% en poids par rapport à la quantité d'eau) |
| Eau déminéralisée | 44,95 |

La composition présente une bonne efficacité déodorante et reste stable après 2 mois de conservation à 4°C, à température ambiante et à 45°C sans formation de cristaux d'alun.

### Exemple 2 : crème déodorante selon l'invention

| **Ingrédients** | **% en poids** |
|---|---|
| Laurylméthicone copolyol vendu par la société Dow Corning Q2- 5200 Formulation Aid | 2,00 |
| Myristate d'isopropyle | 15,00 |
| Alun de potassium vendu par la Société GIULINI CHEMIE (Warwick Chimilux SA) | 10,00 (soit 13,6% en poids par rapport à la quantité d'eau) |
| Eau déminéralisée | 73,00 |

La composition présente une bonne efficacité déodorante et reste stable après 2 mois de conservation à 4°C, à température ambiante et à 45°C sans observer la présence de cristaux d'alun.

### Exemple 3 (comparatif) : Solution aqueuse d'alun à 13,6% en poids

| **Ingrédients** | **% en poids** |
|---|---|
| Alun de potassium vendu par la Société GIULINI CHEMIE (Warwick Chimilux SA) | 13,60 |
| Eau déminéralisée qsp | 100 |

Cette composition comprend l'alun dissous dans la même concentration par rapport à la quantité d'eau (ie : 13,6% en poids) que celle utilisée pour la composition de l'exemple 2. Après 3 jours de conservation à température ambiante, on commence à observer la présence de cristaux d'alun.

### Exemple 4 (comparatif) : Roll-on déodorant émulsion H/E à 10% d'Alun de potassium

| **Ingrédients** | **% en poids** |
|---|---|
| Alcool cétylstéarylique vendu par la société CONDEA sous le nom commercial NAFOL 1618F | 2,5 |
| Alcool cétylstéarylique oxyéthyléné (330E) vendu par la société CONDEA sous le nom commercial EMULDAC AS 25 | 1,25 |
| Monostéarate de sorbitan vendu par la société ICI sous le nom commercial TWEEN 60 | 2,2 |
| Alcool stéarylique vendu par la société AEGIS CHEMICAL sous le nom commercial ACILOL 18 | 0.3 |
| Isohexadécane vendu par la société BAYER | 6 |
| Propylène Glycol vendu par la société BASF | 3 |
| Méthyl paraben vendu par la société UNIPEX | 0.1 |
| Alun de potassium vendu par la Société GIULINI CHEMIE (Warwick Chimilux SA) | 10,15 (soit 13.6% en poids par rapport à la quantité d'eau) |
| Eau déminéralisée | 74,50 |

Cette émulsion continue aqueuse comprend l'alun dissous dans la même concentration par rapport à la quantité d'eau (ie : 13.6% en poids) que celle de la composition de l'exemple 2. Elle est instable. Après 2 mois de conservation à 4°C et à température ambiante, on observe la présence de cristaux d'alun et un déphasage de la formule.

### Exemple 5 : crème déodorante selon l'invention

| **Ingrédients** | **% en poids** |
|---|---|
| Laurylméthicone copolyol vendu par la société Dow Corning Q2- 5200 Formulation Aid | 2,00 |
| Myristate d'isopropyle | 15,00 |
| Alun de potassium vendu par la Société GIULINI CHEMIE (Warwick Chimilux SA) | 15,00 (soit 22% en poids par rapport à la quantité d'eau) |
| Eau déminéralisée | 68,00 |

La composition présente une bonne efficacité déodorante et reste stable après 2 mois de conservation à 4°C, à température ambiante et à 45°C sans formation de cristaux d'alun.

### Exemple 6 (comparatif) : Solution aqueuse d'alun à 22% en poids

| **Ingrédients** | **% en poids** |
|---|---|
| Alun de potassium vendu par la Société GIULINI CHEMIE (Warwick Chimilux SA) | 22,00 |
| Eau déminéralisée qsp | 100,00 |

Cette composition comprend l'alun dissous dans la même concentration par rapport à la quantité d'eau (ie : 22% en poids) que celle de la composition de l'exemple 4. Elle est instable. Après 2 mois de conservation à 4°C et à température ambiante, on observe la présence de cristaux d'alun.

### Exemple 7 (comparatif) : Roll-on déodorant émulsion H/E à 15% d'alun de potassium

| **Ingrédients** | **% en poids** |
|---|---|
| Alcool cétylstéarylique vendu par la société CONDEA sous le nom commercial NAFOL 1618F | 2,5 |
| Alcool cétylstéarylique oxyéthyléné (330E) vendu par la société CONDEA sous le nom commercial EMULDAC AS 25 | 1,25 |
| Monostéarate de sorbitan vendu par la société ICI sous le nom commercial TWEEN 60 | 2,2 |
| Alcool stéarylique vendu par la société AEGIS CHEMICAL sous le nom commercial ACILOL 18 | 0.3 |
| Isohexadécane vendu par la société BAYER | 6 |
| Propylène Glycol vendu par la société BASF | 3 |
| Methyl paraben vendu par la société UNIPEX | 0.1 |
| Alun de potassium vendu par la Société GIULINI CHEMIE (Warwick Chimilux SA) | 15,55 (soit 22% en poids par rapport à la quantité d'eau) |
| Eau déminéralisée | 69,1 |

Cette émulsion continue aqueuse comprend l'alun dissous dans la même concentration par rapport à la quantité d'eau (ie : 22% en poids) que celle de la composition de l'exemple 5. Elle est instable. Après 2 mois de conservation à 4°C et à température ambiante, on observe la présence de cristaux d'alun et un déphasage de la formule.

### Exemple 8 : Composition aérosol selon l'invention

| **Ingrédients de la formulation du jus de la formulation aérosol** | **% en poids** |
|---|---|
| Mélange de diméthicone copolyol vendu par la société Dow Corning Q2- 3225C | 9,1 |
| Cyclopentadiméthylsiloxane vendue par la société Dow Corning sous le nom de Dow Corning 245 fluid | 9,1 |
| Alun de potassium vendu par la Société GIULINI CHEMIE (Warwick Chimilux SA) | 15,00 (soit 22,4% en poids par rapport à la quantité d'eau) |
| Eau déminéralisée | 66,8 |
| | |

| **Composition de la formulation aérosol** | **% en poids** |
|---|---|
| Jus | 45 |
| Isobutane (propulseur) | 55 |

La composition présente une bonne efficacité déodorante et reste stable après 2 mois de conservation à 4°C, à température ambiante et à 45°C sans formation de cristaux d'alun.

**Tableau récapitulatif**

| **Exemple N°** | **1*** | **2*** | **3** | **4** | **5*** | **6** | **7** | **8*** |
|---|---|---|---|---|---|---|---|---|
| **Support** | stick émulsion E/H | émulsion E/H | Solution aqueuse | émulsion H/E | émulsion E/H | solution aqueuse | émulsion H/E | Jus émulsion E/H Aérosol |
| **% Alun en poids / Qté d'eau** | 33.3% | 13.6% | 13.6% | 13.6% | 22% | 22% | 22% | 22.4% |
| **Présence de cristaux d'Alun** | NON | NON | OUI | OUI | NON | OUI | OUI | NON |

| | | | | | | | | |
|---|---|---|---|---|---|---|---|---|
| (*) exemple suivant l'invention | | | | | | | | |

## Revendications

1. Composition déodorante, caractérisée par le fait qu'elle se présente sous la forme d'une émulsion eau-dans-huile contenant une quantité efficace d'au moins un sel d'alun à l'état dissous dans la phase aqueuse de ladite émulsion.

2. Composition selon la revendication 1, où le sel d'alun est choisi dans le groupe constitué par :
- l'alun de potassium de structure : KAl(SO₄)₂, 12H₂O
- l'alun d'ammonium de structure : NH₄Al(SO₄)₂, 12H₂O
- l'alun de sodium de structure : NaAl(SO₄)₂, 12H₂O
- les sels de sulfate d'aluminium de structure : Al₂(SO₄)₃, n H₂O où n vaut 0 à 27

3. Composition selon la revendication 1, où le sel d'alun est choisi parmi les sels d'alun ayant une solubilité inférieure à 30 g/100 ml d'eau à 20°C.

4. Composition selon la revendication 3, où le sel d'alun est choisi parmi les aluns ayant une solubilité inférieure à 13 g/100 ml d'eau à 20°C.

5. Composition selon l'une quelconque des revendications 1 à 4, où le sel d'alun est l'alun de potassium.

6. Composition selon l'une quelconque des revendications 1 à 5, où l'alun est présent à l'état dissous dans la phase aqueuse dans une concentration allant de préférence entre 0,5 à 30% en poids par rapport au poids total de la composition.

7. Composition selon l'une quelconque des revendications 1 à 6, où l'alun est présent à l'état dissous dans la phase aqueuse dans une concentration supérieure à 10% en poids par rapport au poids total de la composition.

8. Composition selon l'une quelconque des revendications 1 à 7, où l'alun est présent à l'état dissous dans la phase aqueuse dans une concentration supérieure ou égale à 15% en poids par rapport au poids total de la composition.

9. Composition selon l'une quelconque des revendications 1 à 8, où la phase grasse est constituée par une ou plusieurs huiles et/ou une ou plusieurs cires.

10. Composition selon la revendication 9, où les huiles sont choisies parmi les huiles minérales, les huiles végétales, les huiles synthétiques, les huiles fluorées ou perfluorées, les polyalkylènes, les huiles de silicone volatiles ou non volatiles.

11. Composition selon la revendication 9, où les cires sont choisies parmi les cires animales, fossiles, végétales, minérales ou de synthèse, l'huile de ricin hydrogénée, les paraffines, les cires et résines de silicone.

12. Composition selon l'une quelconque des revendications 1 à 11, où la phase grasse est présente dans les compositions dans des concentrations pondérales allant de 5% à 80% par rapport au poids total de la composition, et de préférence dans des concentrations pondérales allant de 10% à 50% par rapport au poids total de la composition.

13. Composition selon l'une quelconque des revendications 1 à 12, où la phase aqueuse peut contenir en outre des humectants, des stabilisants, des séquestrants.

14. Composition selon l'une quelconque des revendications 1 à 13, caractérisée par le fait qu'elle contient en plus un ou plusieurs tensioactifs émulsionnants E/H ayant un HLB inférieur ou égal à 6.

15. Composition selon la revendication 14, où le ou les tensioactifs émulsionnants E/H sont choisis parmi les esters d'acide gras et de glucose ; les esters d'acide gras et de glycérine ; les esters d'acide gras et de sorbitol ; les polyalkyl polyéther siloxanes portant des groupes polyoxyalkylénés greffés sur la chaîne siliconée principale.

16. Composition selon la revendication 15, où les tensioactifs émulsionnants E/H sont choisis dans le groupe formé par les polydiorganosiloxanes de formules (I) et (II) suivantes, ces composés pouvant eux-mêmes être dispersés dans une diméthicone volatile, 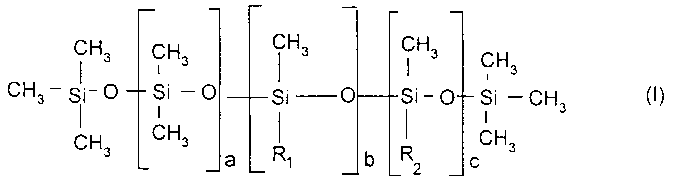 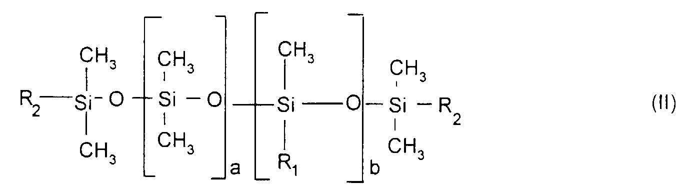 dans lesquelles :
R₁ désigne un groupement alkyle linéaire ou ramifié en C₁₂-C₂₀ et de préférence en C₁₂-C₁₈ ;
R₂ désigne le groupement :--CₙH₂ₙ--(-OC₂H₄-)ₓ--(-OC₃H₆-)_{y}--O―R₃,
R₃ désigne un atome d'hydrogène ou un radical alkyle linéaire ou ramifié comportant de 1 à environ 12 atomes de carbone,
a désigne un nombre entier allant de 0 à environ 500,
b est un nombre entier allant de 0 à environ 500
c désigne un nombre entier allant de 1 à environ 500,
n est un nombre entier allant de 2 à 12,
x et y désignent respectivement un nombre entier allant de 0 à environ 50, la somme x + y étant supérieure ou égale à la valeur 1.

17. Composition selon la revendication 16, où l'émulsionnant E/H est un polymère de formule (I) dans laquelle, a est un nombre entier allant de 2 à 450, b vaut 0, c est un nombre entier allant de 2 à 40, n est un nombre entier allant de 2 à 5, x est un nombre entier allant de 1 à 30, y est un nombre entier allant de 0 à 30, avec x ≥ y.

18. Composition selon la revendication 17, où l'émulsionnant E/H est un mélange de cyclométhicone et de diméthicone copolyol (nomenclature C.T.F.A.)

19. Composition selon la revendication 16, où l'émulsionnant E/H est un polymère de formule (I) dans laquelle a vaut 0 et b est différent de 0.

20. Composition selon la revendication 19, où l'émulsionnant E/H est le lauryl méthicone copolyol ou le cétyl diméthicone copolyol (nomenclature CTFA)

21. Composition selon l'une quelconque des revendications 14 à 18, où le ou les tensioactifs émulsionnants E/H sont présents dans des proportions pondérales allant d'environ 0,1% à environ 10%, et de préférence allant d'environ 1% à environ 8% par rapport au poids total de la composition.

22. Composition selon l'une quelconque des revendications 1 à 19, caractérisée par le fait qu'elle contient en plus d'autres actifs déodorants en plus de l'alun.

23. Composition selon la revendication 22, où l'actif déodorant additionnel est choisi parmi les sels d'aluminium et/ou les sels de zirconium, le bicarbonate de sodium, les agents bactériostatiques, les agents bactéricides, les substances absorbant les odeurs et les agents antioxydants.

24. Composition selon l'une quelconque des revendications 1 à 23, caractérisée par le fait qu'elle contient en plus des adjuvants cosmétiques choisis parmi les solvants organiques, les gélifiants, les émollients, les adoucissants, les antioxydants, les opacifiants, les stabilisants, les silicones, les agents anti-mousse, les agents hydratants, les vitamines, les parfums, les conservateurs, les tensioactifs, les charges, les polymères, les propulseurs, les agents alcalinisants ou acidifiants, , les colorants, les pigments, les agents épaississants, ou tout autre ingrédient habituellement utilisé en cosmétique.

25. Composition selon l'une quelconque des revendications 1 à 24 , caractérisée par le fait qu'elle se présente sous forme d'une lotion, d'une crème ou d'un gel fluide distribué en spray aérosol, en flacon pompe ou en roll-on ; sous forme d'une crème épaisse distribuée en tubes ; ou sous forme de stick.

26. Formulation cosmétique ou dermatologique déodorante , caractérisée par le fait qu'elle est constituée d'une composition selon l'une quelconque des revendications 1 à 25.

27. Utilisation d'une composition selon l'une quelconque des revendications 1 à 25 pour la fabrication de produits cosmétiques ou dermatologiques destinés à traiter les odeurs axillaires humaines.

28. Procédé pour traiter les odeurs axillaires humaines, consistant à appliquer sur la surface axillaire une quantité efficace d'une composition selon l'une quelconque des revendications 1 à 24.

29. Utilisation d'une composition selon l'une quelconque des revendications 1 à 25 pour la fabrication de produits d'entretien et désodorisants.

30. Utilisation d'un support du type émulsion eau-dans-huile pour la fabrication de produits cosmétiques ou dermatologiques déodorants aqueux contenant un sel d'alun à l'état dissous.

31. Utilisation selon la revendication 30, où le sel d'alun est présent à l'état dissous dans la phase aqueuse dans une concentration supérieure à 10% en poids par rapport au poids total de la composition.

32. Utilisation selon la revendication 30, où le sel d'alun est présent à l'état dissous dans la phase aqueuse dans une concentration supérieure ou égale à 15% en poids par rapport au poids total de la composition.
